(19) Europäisches Patentamt
European Patent Office
Office européen des brevets

(11) Publication number : **0 309 541 B1**

(12) # EUROPEAN PATENT SPECIFICATION

(45) Date of publication of patent specification : **02.01.92 Bulletin 92/01**

(51) Int. Cl.$^5$ : **A61K 31/47**, C07D 215/12, C07D 215/14, C07D 215/22, C07D 215/36, C07D 215/38, C07D 215/48

(21) Application number : **88903531.7**

(22) Date of filing : **16.03.88**

(86) International application number : **PCT/US88/00767**

(87) International publication number : **WO 88/06886 22.09.88 Gazette 88/21**

(54) **SULFONYLCARBOXAMIDES.**

The file contains technical information submitted after the application was filed and not included in this specification

(30) Priority : **18.03.87 US 27452**

(43) Date of publication of application : **05.04.89 Bulletin 89/14**

(45) Publication of the grant of the patent : **02.01.92 Bulletin 92/01**

(84) Designated Contracting States : **AT BE CH DE FR IT LI LU NL SE**

(56) References cited :
**EP-A- 0 200 101**
**EP-A- 0 219 308**
**EP-B- 206 751**
**EP-B-18 156 8**
**GB-A- 1 079 236**
**US-A- 4 314 065**
**US-A- 4 444 584**
**US-A- 4 581 457**

(56) References cited :
**US-A- 4 594 425**
**US-A- 4 631 287**
**US-A- 4 675 405**
**US-A- 4 684 394**
**US-A- 4 754 043**

(73) Proprietor : **AMERICAN HOME PRODUCTS CORPORATION**
**685, Third Avenue**
**New York, New York 10017 (US)**

(72) Inventor : **KREFT, Anthony, F. III**
**512 Lexington Lane**
**Trooper, PA 19403 (US)**
Inventor : **MUSSER, John, H.**
**1009 Millstream Drive**
**Malvern, PA 19355 (US)**
Inventor : **KUBRAK, Dennis, M.**
**856 Eaton Road**
**Drexel Hill, PA 19026 (US)**

(74) Representative : **Wileman, David Francis et al**
**c/o John Wyeth and Brother Limited**
**Huntercombe Lane South**
**Taplow Maidenhead Berkshire SL6 OPH (GB)**

EP 0 309 541 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

This invention relates to novel sulfonylcarboxamide compounds possessing lipoxygenase inhibitory and leukotriene antagonist activity, which are useful as anti-inflammatory and antiallergic agents.

It is known that arachidonic acid (AA) is metabolized in mammals by two distinct pathways. The metabolism of arachidonic acid by cyclooxygenase enzymes results in the production of prostaglandins and thromboxanes. The physiological activity of the prostaglandins has already been amply elucidated in recent years. The other pathway of AA metabolism involves lipoxygenase enzymes and results in the production of a number of oxidative products called leukotrienes. The latter are designated by the LT nomenclature system, and the most significant products of the lipoxygenase metabolic pathway are the leukotrienes $B_4$, $C_4$, $D_4$ and $E_4$. The substance denominated slow-reacting substance of anaphylaxis (SRS-A) has been shown to consist of a mixture of sulfidopeptide leukotrienes, $C_4$, $D_4$ and $E_4$ [see Bach et al.. J. Immun. 215, 115-118 (1980); Biochem. Biophys. Res. Commun. 93, 1121-1126 (1980)].

The significance of these leukotrienes is that a great deal of evidence has been accumulated showing that leukotrienes participate in inflammatory reactions, exhibit chemotactic activities, stimulate lysosomal enzyme release and act as important factors in the immediate hypersensitivity reaction. It has been shown that $LTC_4$ and $LTD_4$ are potent bronchoconstrictors of the human bronchi [see Dahlen et al., Nature 288, 484-486 (1980) and Piper, Int. Arch. Appl. Immunol., 76, suppl. 1, 43 (1985)] which stimulate the release of mucus from airways in vitro [Marom et al., Am.Rev. Resp. Dis., 126, 449 (1982)], are potent vasodilators in skin [see Bisgaard et al, Prostaglandins, 23, 797 (1982)], and produce a wheal and flare response [Camp et al., Br. J. Pharmacol., 80, 497 (1983)]. The nonpeptide leukotriene, $LTB_4$, is a powerful chemotactic factor for leukocytes [see A.W. Ford-Hutchinson, J. Roy. Soc. Med., 74, 831-833 ( 1981) ], which stimulates cell accumulation and affects vascular smooth muscle [see Bray, Br. Med. Bull., 39, 249 (1983)]. The activity of leukotrienes as mediators of inflammation and hypersensitivity is extensively reviewed in Bailey and Casey, Ann. Reports Med. Chem., 17, 203-217 (1982) and in Bray, Agents and Actions, 19, 87 (1986).

Accordingly, the biological activity of the leukotrienes and SRS's, and of lipoxygenase as the enzyme leading to the metabolism of AA to leukotrienes, indicates that a rational approach to drug therapy to prevent, remove or ameliorate the symptoms of allergies, anaphylaxis, asthma and inflammation must focus on either blocking the release of mediators of these conditions or antagonizing their effects. Thus compounds, which inhibit the biological effects of the leukotrienes and SRS's and/or which control the biosynthesis of these substances, as by inhibiting lipoxygenase, are considered to be of value in treating such conditions as allergic bronchial asthma, allergic rhinitis, as well as in other immediate hypersensitivity reactions.

It has now been found that certain novel sulfonylcarboxamide compounds antagonize products of the lipoxygenase pathway, and so are useful as anti-inflammatory and anti-allergic agents. The present invention provides novel compounds having the following formula:

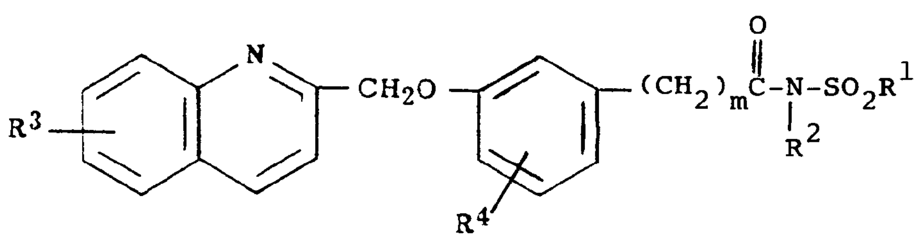

wherein

$R^1$ is phenyl substituted with $R^5$;

$R^2$ is hydrogen or lower alkyl;

$R^3$, $R^4$ and $R^5$ are each independently hydrogen, lower alkyl, nitro, trifluoromethyl, halo, lower alkoxy, lower alkoxycarbonyl or lower alkanoyloxy;

m is 0 - 10;

and the pharmaceutically acceptable salts thereof.

The terms "lower alkyl" and "lower alkoxy" refer to moieties having 1 to 6 carbons atoms in the carbon chain, the term "lower alkanoyloxy" refers to moieties of the structure RC00- wherein R is lower alkyl as defined hereinbefore. The term "halo" refers to fluoro, chloro and bromo.

This invention also provides processes for preparing the compounds of formula I.

A first general process for preparing the compounds of formula I comprises reacting a compound of formula

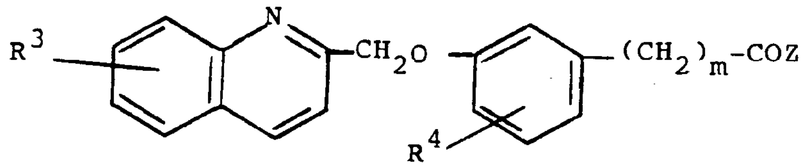

(II)

wherein m, X, $R^3$ and $R^4$ are as defined above and Z is OH or a reactive derivative thereof with a sulphonamide of formula

$$HNR^2SO_2R^1 \qquad (III)$$

wherein $R^1$ and $R^2$ are as defined above.

Examples of reactive derivatives of the acid are the acid halide, eg. chloride or bromide, mixed anhydride (eg. formed from carbonyldiimidazole), azide, activated ester (eg. 1-benzotriazolyl, 2,4,5-trichlorophenyl or p-nitrophenyl activated esters) or 0-acyl urea obtained from carbodiimides, such as dialkylcarbodiimides, eg. dicyclohexylcarbodiimide. Descriptions of methods for activating carboxy groups are given in general textbooks of peptide chemistry, eg. The Practise of Peptide Synthesis, by M Bodanszky and A Bodanszky Springer-Verlaz 1984, Volume 21 of the series "Reactivity and Structure Concepts in Organic Chemistry".

The starting materials of formula II wherein Z is OH can be prepared by reacting a compound of formula:

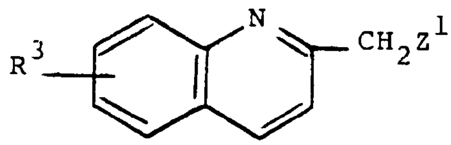

(IV)

wherein $R^3$ is as defined above and $Z^1$ is a halogen eg. chlorine or an organic sulphonyloxy radical with a compound of formula:

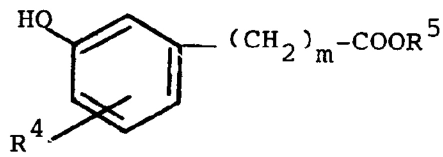

(V)

wherein m and $R^4$ are as defined above, and $R^5$ is hydrogen when m is $\geqq 2$ otherwise $R^5$ is lower alkyl, and when $R^5$ is lower alkyl, hydrolysing the product. The reaction is conveniently carried out in the presence of base e.g

NaOMe when $R^5$ is hydrogen or $CsC0_3$ when $R^5$ is lower alkyl

A second process for preparing compounds of formula I comprises reacting a compound of formula IV as defined above with a compound of formula

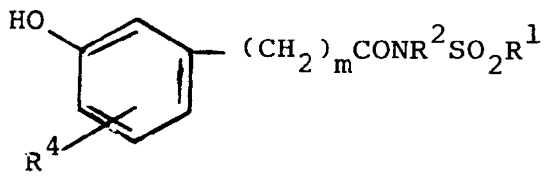

(Va)

in which m,$R^1$, $R^2$ and $R^4$ are as defined above. The reaction is conveniently carried out in the presence of base, e.g an alkali metal carbonate such as caesium carbonate. Example of $Z^1$ in the compound of formula IV are chlorine, tosyloxy or mesyloxy.

In a preferred embodiment the compounds of the invention in which $m \geqq 2$ can be prepared according to the following representative sequence.

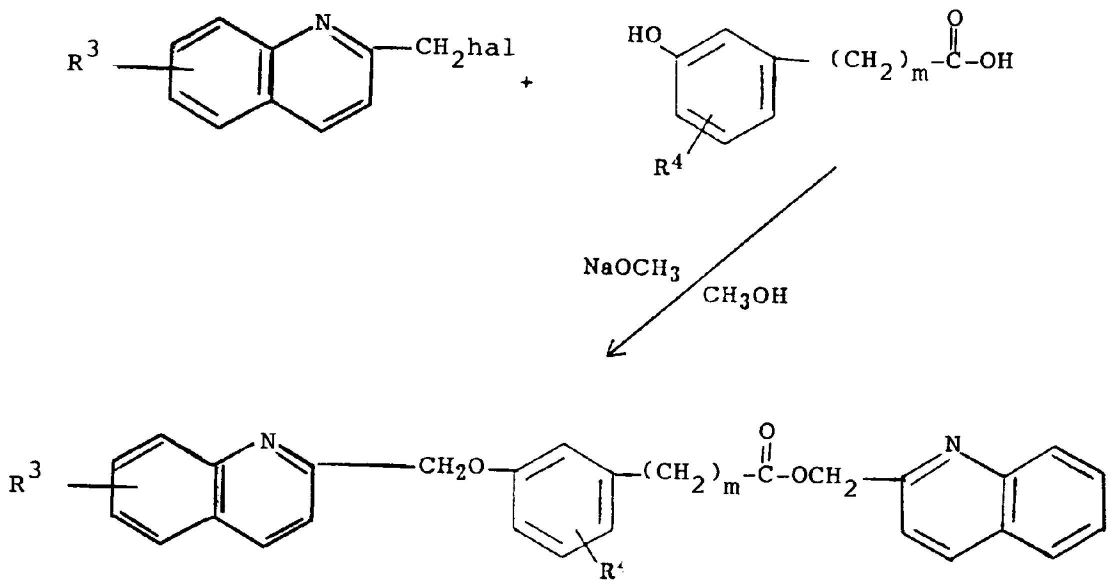


,

The resulting compounds obtained by this sequence are hydrolyzed to yield intermediate carboxylic acids:

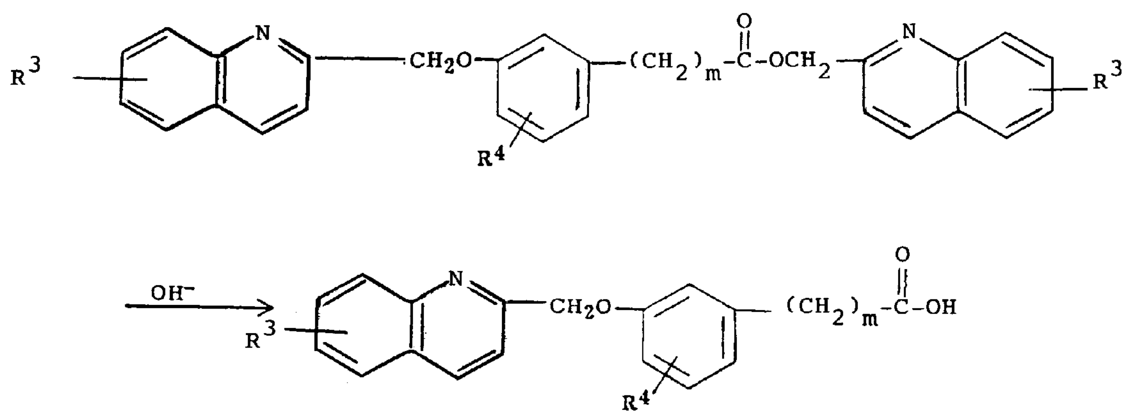


which are then reacted with an appropriate sulfonamide reactant to yield the desired sulfonylcarboxamide derivatives:

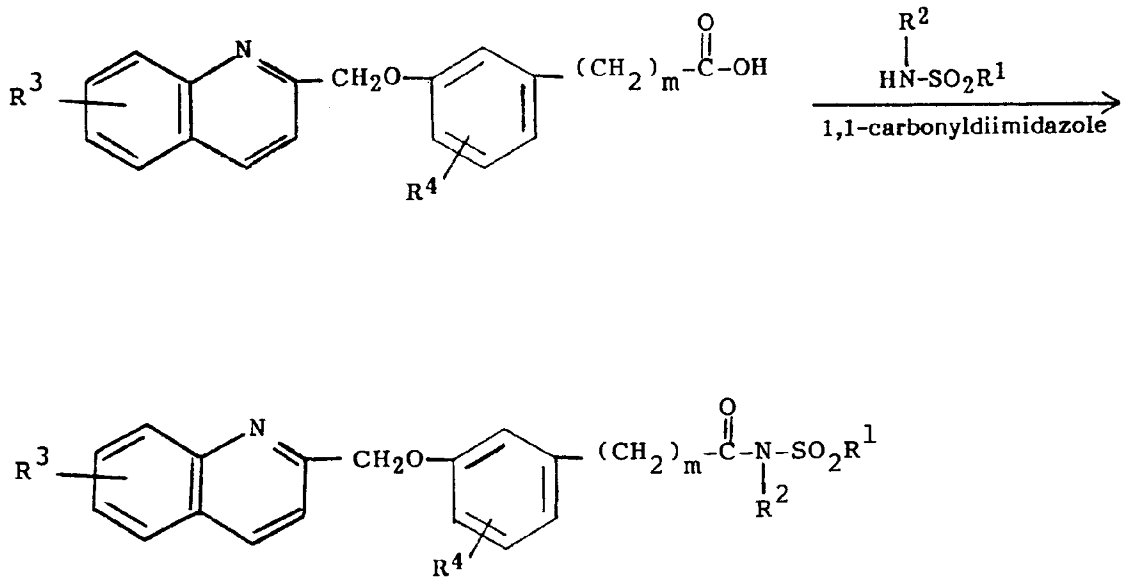


Compounds in which m is 0 or 1 can be prepared according to a preparative sequence employing a hydroxy acid ester as the starting compound and which is carried out as follows:

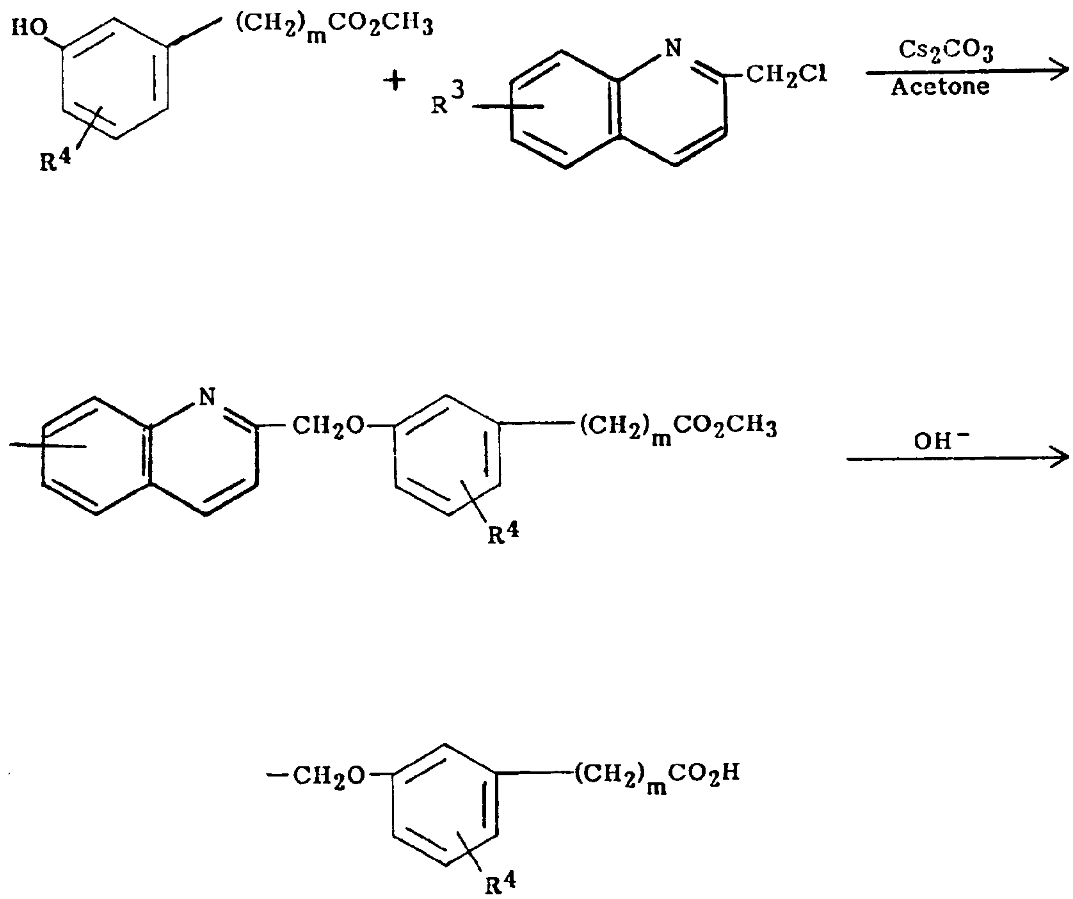


The resulting carboxylic acid intermediate can then be reacted as in the first described reaction sequence to obtain the desired final product sulfonylcarboxamides.

The benzo-fused heterocyclic compounds used as starting material in the above reaction sequence are

either commercially available or can be prepared by methods conventional in the art.

Compounds of the invention which contain a basic nitrogen are capable of forming pharmaceutically acceptable salts, including the salts of pharmaceutically acceptable organic and inorganic acids such as hydrochloric, hydrobromic, sulfuric, nitric, phosphoric, methanesulfonic, benzenesulfonic, acetic, citric, fumaric, maleic, succinic and the like.

The compounds of the invention, by virtue of their ability to inhibit the activity of lipoxygenase enzyme and to antagonize mediators arising from this enzymatic pathway, are useful in the treatment of inflammatory conditions. Accordingly, the compounds are indicated in the treatment of such diseases as rheumatoid arthritis, osteoarthritis, tendinitis, bursitis and similar conditions involving inflammation. Moreover, by virtue of their ability to inhibit the activity of lipoxygenase enzyme and by their ability to antagonize the effect of $LTC_4$, $LTD_4$ and $LTE_4$ which are the constituents of SRS-A, they are useful for the inhibition of symptoms induced by these leukotrienes. Accordingly, the compounds are indicated in the prevention and treatment of those disease states in which $LTC_4$, $LTD_4$ and $LTE_4$ are causative factors, for example allergic rhinitis, allergic bronchial asthma and other leukotriene mediated naso-bronchial obstructive air-passageway conditions, as well as in other immediate hypersensitivity reactions, such as allergic conjunctivitis. The compounds are especially valuable in the prevention and treatment of allergic bronchial asthma.

When the compounds of the invention are employed in the treatment of allergic airway disorders and/or as antiinflammatory agents, they can be formulated into oral dosage forms such as tablets, capsules and the like. The compounds can be administered alone or by combining them with conventional carriers, such as magnesium carbonate, magnesium stearate, talc, sugar, lactose, pectin, dextrin, starch, gelatin, tragacanth, methylcellulose, sodium carboxymethylcellulose, low melting wax, cocoa butter and the like. Diluents, flavoring agents, solubilizers, lubricants, suspending agents, binders, tablet-disintegrating agents and the like may be employed. The compounds may be encapsulated with or without other carriers. In all cases, the proportion of active ingredients in said compositions both solid and liquid will be at least to impart the desired activity thereto on oral administration. The compounds may also be injected parenterally, in which case they are used in the form of a sterile solution containing other solutes, for example, enough saline or glucose to make the solution isotonic. For administration by inhalation or insufflation, the compounds may be formulated into an aqueous or partially aqueous solution, which can then be utilized in the form of an aerosol.

The dosage requirements vary with the particular compositions employed, the route of administration, the severity of the symptoms presented and the particular subject being treated. Treatment will generally be initiated with small dosages less than the optimum dose of the compound. Thereafter the dosage is increased until the optimum effect under the circumstances is reached. In general, the compounds of the invention are most desirably administered at a concentration that will generally afford effective results without causing any harmful or deleterious side effects and can be administered either as a single unit dose, or if desired, the dosage may be divided into convenient subunits administered at suitable times throughout the day.

The lipoxygenase and cyclooxygenase inhibitory and leukotriene antagonist effects as well as the antiinflammatory effects of the compounds of the invention may be demonstrated by standard pharmacological procedures, which are described more fully in the examples given hereinafter.

These procedures illustrate the ability of the compounds of the invention to inhibit the polymorphonuclear leukocyte synthesis of the lipoxygenase product 5-HETE and the cyclooxygenase product $PGE_2$; the in vivo ability of the compounds to inhibit bronchospasm induced by exogenously administered mediators of bronchoconstriction; and measure the in vivo activity of the compounds as antiinflammatory agents in the rat carrageenan paw edema assay.

The following examples show the preparation and pharmacological testing of compounds within the invention.

**Example 1**

**N-[(4-Methylphenyl)sulfonyl]-3-(2-quinolylmethoxy)benzenepropanamide**

A. To a solution of 3-p-(hydroxyphenyl)propionic acid (20.0 g, 0.12 mol) in methanol (50 ml) is added dropwise sodium methoxide in methanol (55 ml, 25 wt. % solution). After stirring 1 hour, the methanol is evaporated in vacuo, dimethylformamide (150 ml) and 2-chloromethylquinoline (42.6 g, 0.12 mol) are added thereto, and the mixture is stirred at room temperature for 48 hours. The dimethylformamide is removed in vacuo, methylene chloride is added and the organic phase is washed with water, dried ($MgSO_4$), filtered and concentrated to an oil. The oil is purified by HPLC using hexane : acetone as eluent. The viscous oil isolated from fractions 12 - 19, is triturated with isopropyl ether to produce 16.85 g of crystalline solid, m.p. 73-76°C (31% yield).

B. The dialkylated product obtained in step A. above is hydrolyzed to the carboxylic acid as follows: A mixture of the product of step A. (16.8 g, 37.0 mmol) in tetrahydrofuran and 1 N sodium hydroxide (125 ml) is refluxed for 3 hours. The tetrahydrofuran is removed in vacuo and the aqueous layer washed 2 times with methylene chloride. The aqueous layer is acidified using 1 N HCl solution. Filtration and drying produces 11.3 g white solid, m.p. 130-132°C (quantitative yield).

C. To a solution of the 3-[(2-quinolinylmethoxy)]phenylpropionic acid obtained in step B. above (1.7 g, 5.5 mmol) in tetrahydrofuran (50 ml) is added 1,1-carbonyldiimidazole (0.9 g, 5.5 mmol) in tetrahydrofuran. After 1 hour, p-toluene-sulfonamide (0.94 g, 5.5 mmol) is added to the reaction mixture. After overnight stirring, the mixture is concentrated to an oil, and the oil is purified by HPLC using ethyl acetate as eluent. The material is then crystalized with diisopropyl ether/ethyl ether to give 0.78 g of white crystalline solid, m.p. 136-138°C (31% yield).

| | |
|---|---|
| Analysis for: | $C_{26}H_{24}N_2O_4S$ |
| Calculated: | C, 67.80; H, 5.25; N, 6.08 |
| Found: | C, 68.00; H, 5.27; N, 6.00. |

**Example 2**

**N-[(4-Methylphenyl)sulfonyl]-3-(2-quinolinylmethoxy)benzene ethanamide**

A. To a solution of 3-hydroxyphenyl acetic acid methyl ester (14.1 g, 85 mmol) in 300 ml acetone is added 2-chloromethyl quinoline (15.09 g 85 mmol) cesium carbonate (29 g, 89 mmol) and potassium iodide (0.16 g, 1 mmol). The mixture is refluxed 40 hours, filtered through a pad of Celite and silica gel and the solvent removed in vacuo. This gives the product as an oil (25.4 g, 97% yield).

| | |
|---|---|
| Analysis for: | $C_{19}H_{17}NO_3 \cdot 0.1\ H_2O$ |
| Calculated: | C, 73.81; H, 5.60; N, 4.53 |
| Found: | C, 73.76; H, 5.51; N, 4.53. |

B. The ester of A. above is transformed into the carboxylic acid as follows: To a solution of the ester (19.5 g, 63.4 mmol) in tetrahydrofuran (150 ml) is added 1 N NaOH solution (150 ml) and the mixture is refluxed for 3 hours. The tetrahydrofuran is removed in vacuo, the aqueous phase acidified with 1 N HCl solution and the solid filtered and dried to give 17.5 g (94% yield) m.p. 128-130°C.

| | |
|---|---|
| Analysis for: | $C_{18}H_{15}NO_3 \cdot 1/4\ H_2O$ |
| Calculated: | C, 72.59; H, 5.16; N, 4.70 |
| Found: | C, 72.90; H, 5.23; N, 4.90. |

C. To a solution of the acid of step B. above (2.6 g, 8.8 mmol) in tetrahydrofuran (50 ml) is added 1,1-carbonyldiimidazole (1.44 g, 8.8 mmol) in tetrahydrofuran. After 30 minutes, p-toluene-sulfonamide (1.5 g, 8.8 mmol) is added to the reaction mixture, then successively stirred overnight at room temperature, overnight at reflux, then (after tetrahydrofuran removal) overnight at reflux in toluene. The mixture is concentrated and purified by HPLC using ethyl acetate as eluent. Finally the material is recrystallized from ethyl acetate producing 0.7 g of tan amorphous solid (18% yield), m.p. 174-176° C.

| | |
|---|---|
| Analysis for: | $C_{25}H_{22}N_2O_4S$ |
| Calculated: | C, 67.24; H, 4.96; N, 6.27 |
| Found: | C, 66.89; H, 4.93; N, 6.07. |

**Example 3**

**N-[(4-Methylphenyl)sulfonyl]-3-(2-quinolinylmethoxy)benzamide**

A. To a solution of methyl-3-hydroxybenzoate (7.6 g, 0.05 mol) in 200 ml acetone is added 2-chloromethylquinoline (8.88 g, 0.05 mol), cesium carbonate (16.3 g, 0.05 mol) and potassium iodide (0.16 g, 1 mmol). The mixture is refluxed overnight, filtered through Celite and silica gel and the solvent removed in vacuo. This produces an oil which crystallizes from ethanol to give 12.3 g of crystalline solid (83% yield) m.p. 55-57° C.

B. The ester of step A. above is hydrolyzed by adding 1 N NaOH (80 ml) to the ester [5.7 g, 19.4 mmol) in tetrahydrofuran (80 ml), and refluxing overnight. The tetrahydrofuran is removed in vacuo, the aqueous phase is acidified with 1 N HCl solution and the solid filtered and dried to give 5.4 g (quantitative yield) m.p. 180-184°C.

| | |
|---|---|
| Analysis for: | $C_{17}H_{13}NO_3 \cdot 1/2\ H_2O$ |
| Calculated: | C, 70.82; H, 4.89; N, 4.85 |

Found: C, 70.42; H, 4.61; N, 4.92.

C. To a solution of 3-[(2-quinolinylmethyl)oxy]benzoic arid of step B. above (7.6 g, 27 mmol) in tetrahydrofuran (150 ml) is added 1,1-carbonyldiimidazole (4.44 g, 27 mmol) in tetrahydrofuran. After 1 hour, p-toluene-sulfonamide (4.6 g, 27 mmol) is added to the reaction mixture. After overnight stirring, the mixture is filtered and concentrated to an oil; the oil is purified by HPLC eluting with ethylacetate : hexane and finally recrystallized from ethyl acetate to afford 0.97 g of a white solid (14% yield), m.p, 183-185°C.

Analysis for: $C_{24}H_{20}N_2O_4S$
Calculated: C, 66,65; H, 4.66; N, 6.47
Found: C, 66.21; H, 4.55; N, 6.60.

## Example 4

The compounds 5- and 12-hydroxyeicosatetraenoic acid (5-HETE and 12-HETE) and 5,12-dihydroxyeicosatetraenoic acid (5,12-diHETE) are early arachidonic acid oxidation products in the lipoxygenase cascade, which have been shown to mediate several aspects of inflammatory and allergic response. The assay of this Example measures the ability of the compounds of the invention to inhibit the synthesis of 5-HETE by rat glycogen elicited polymorphonuclear leukocytes.

The assay is carried out as follows:

Peritoneal PMN are obtained from female Wistar rats (150-250 g) that received an i.p. injection of 6% glycogen (10 ml). After 24 hours, rats are killed by $Co_2$ asphyxiation and peritoneal cells are harvested by peritoneal lavage using $Ca^{++}$ and $Mg^{++}$ free Hanks' balanced salt solution (HBSS). The peritoneal exudate is centrifuged at 400 g for 10 minutes. After centrifugation, the lavaged fluid is removed and the cell pellet is resuspended in HBSS containing $Ca^{++}$ and $Mg^{++}$ and 10 mM L-cysteine at a concentration of 2 x $10^7$ cells/ml. To 1 ml portions of cell suspension, test drugs or vehicle are added and incubated at 37°C for 10 minutes. Following this preincubation, the calcium ionophore (10 μM), A23187, is added together with 0.5 μCi $[_{14}C]$ arachidonic acid and further incubated for 10 minutes. The reaction is stopped by the addition of ice cold water (3 ml) and acidifying to pH 3.5. Lipoxygenase products are then extracted twice into diethyl ether. The pooled ether extracts are evaporated to dryness under nitrogen and the residue is redissolved in a small volume of methanol and spotted on aluminum backed pre-coated thin layer chromatographic plates. The samples are then cochromatographed with authentic reference 5-HETE in the solvent system - hexane : ether : acetic acid (50:50:3). After chromatography, the areas associated with 5-HETE standard are identified by autoradiography, cut out and quantitated by liquid scintillation.

The compound of Example 3, when tested in this assay, exhibited an $IC_{50}$ value of 6 μM, evidencing a significant activity in inhibiting the synthesis of the arachidonic acid ligoxygenase oxidation product 5-HETE.

## Example 5

The procedure of Example 4 is also employed for the determination of the ability of the compounds of the invention to inhibit the synthesis of the arachidonic acid cyclooxygenase oxidation product $PGE_2$.

In this assay, the procedure of Example 4 is carried out as described. However, in order to determine cyclooxygenase activity, the samples are cochromatographed with authentic reference $PGE_2$ in the solvent system ethyl acetate : formic acid (80:1) and the upper phase of ethyl acetate : isooctane : acetic acid : water (110:50:20:100). After chromatography, the areas associated with the $PGE_2$ standard are identified by autoradiography, cut out and quantitated by liquid scintillation techniques.

The results are calculated as in Example 4.

When the compound of Example 3 was tested in this assay, it exhibited an $IC_{50}$ value of 13 μM showing that this compound has quite significant activity in inhibiting the synthesis of the arachidonic acid cyclooxygenase oxidation product $PGE_2$.

## Example 6

The assay of this Example measures the in vivo ability of the compounds of the invention to inhibit the bronchospasm induced in guinea pigs by the exogenously administered leukotrienes $C_4$ and/or $D_4$.

This assay is carried out as follows:

Male Hartley strain guinea pigs (350-600g) are anesthetized with pentobarbital sodium (50 mg/kg, i.p.). The jugular vein is cannulated for injection of drugs and the carotid artery for monitoring blood pressure. The trachea is cannulated for artificial ventilation by a miniature Starling pump and for indirect measurement of respiratory volume changes. The animals are then pretreated with succinylcholine (2 mg/kg i.v.) and indomethacin (10

mg/kg i.v. in trizma 8.3 buffer, 9 minutes prior to leukotriene challenge). Submaximal bronchoconstrictor responses are established in control animals by varying the dose-levels of leukotriene. Intravenous dose-levels for $LTC_4$ range from 0.4 to 0.6 µg/kg and for $LTD_4$ the range is from 0.3 to 0.5 µg/kg. The aerosol bronchoprovocation dose for $LTC_4$ is generated from 1.6 µM solution and for $LTD_4$ from a 2.0 µM solution.

Test drugs (dissolved in a solvent such as propylene glycol, polyethylene glycol 400 or saline) are administered either intraduodenally, by aerosol or intragastrically at 2 or 10 minutes before induction of bronchospasm by administration of either $LTC_4$ or $LTD_4$ at the predetermined dose-level. Aerosol of soluble drugs or leukotrienes are produced in-line for 10 seconds only by actuation of an ultrasonic nebulizer (Monaghan). Aerosolized drug dosage is expressed in terms of solution concentration and by a fixed aerosol exposure time (approximately 10 seconds). Control animal receive solvent (2 ml/kg i.d. or appropriate aerosol) in place of drug.

Respiratory volume changes are determined by a calibrated piston whose travel is recorded, via a linear transducer, on a Beckman Dynograph recorder. Maximal bronchoconstrictor volume is determined by clamping off the trachea at the end of the experiment. Overflow volumes at 1, 3 and 5 minutes are obtained from the recorded charts.

Area under the volume overflow curve (AUC) is estimated, using the overflow values at 1, 3 and 5 minutes, and expressed as a percentage of the maximal overflow AUC (equation 1):

$$\% \text{ max AUC} = \frac{3(1 \text{ min}) + 4(3 \text{ min}) + 2(5 \text{ min})}{10(\text{max})} \text{ X } 100 \quad (1$$

Drug effects are reported as percent inhibition of % max AUC values obtained from appropriate control animal (equation 2):

$$\% \text{ inhibition} = \frac{\% \text{ max AUC control} - \% \text{ max AUC treated}}{\% \text{ max AUC control}} \text{ X } 100 \quad (2$$

Student's t-test for unpaired data is used to determine statistical significance ($p < 0.05$). $IC_{50}$ values can also be determined by inverse prediction from linear regression lines through points between 10 and 90% inhibition.

The results for compounds of the invention are as follows:

Compound administered at 10 minutes before induction of bronchospasm

| Compound of Example Number | Dose mg/kg | % Inhibition |
|---|---|---|
| 1 | 25* | 97 |
| | 25** | 66 |
| 2 | 25* | 75 |
| | 25** | 16 |
| 3 | 25* | 97 |
| | 25** | 91 |

* = intraduodenally administered

** = intragastrically administered

The results show that compounds of the invention have significant in vivo activity against $LTD_4$ induced bronchoconstriction.

**Example 7**

The compounds of the invention are further tested in the rat carrageenan paw edema assay to determine their ability to inhibit the acute inflammatory response.

This assay is carried out as follows:

140-180 gm male Sprague-Dawley rats, in groups of 6 animals are injected subcutaneously in the right paw with 0.1 ml of 1% carrageenan at zero time. Mercury plethysmographic readings (ml) of the paw are made at zero time and 3 hours later. Test compounds are suspended or dissolved in 0.5% methylcellulose and given perorally 1 hour prior to carrageenan administration.

The increase in paw volume (edema in ml.) produced by the carrageenan is measured. Paw edema is calculated (3 hour volume minus zero time volume), and percent inhibition of edema is determined. Unpaired Student's t-test is used to determine statistical significance.

The activity of standard drugs in this assay is as follows:

| Drug | Oral $ED_{50}$ (95% C.L.) mg/kg |
| --- | --- |
| Indomethacin | 3.7 (0.6, 23.8) |
| Aspirin | 145.4 (33.1, 645.6) |
| Phenylbutazone | 26.2 (2.3, 291.0) |

When tested in this assay, the compounds of the invention gave the following results:

| Compound of Example No. | % Inhibition at 50 mg/kg (peroral) |
| --- | --- |
| 1 | 40 |
| 2 | 42 |
| 3 | 50 |

The results show that the compounds tested have activity in the rat carrageenan paw edema assay, evidencing an effect on the acute inflammatory response.

## Claims

1. A compound having the formula:

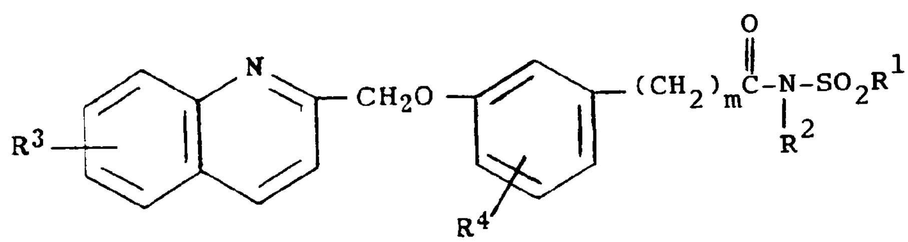

(I)

wherein

$R^1$ is phenyl substituted with $R^5$;

$R^2$ is hydrogen or $C_1$-$C_6$ alkyl;

$R^3$, $R^4$ and $R^5$ are each independently hydrogen, $C_1$-$C_6$ alkyl, nitro, trifluoromethyl, halo, $C_1$-$C_6$ alkoxy, ($C_1$-$C_6$ alkoxy)carbonyl or $C_2$-$C_7$ alkanoyloxy;

m is 0 - 10;

or a pharmaceutically acceptable salt thereof.

2. A compound as claimed in Claim 1 wherein m is 0, 1 or 2.

3. N-[(4-Methylphenyl)sulfonyl]-3-(2-quinolinylmethoxy)benzenepropanamide or a pharmaceutically acceptable salt thereof.

4. N-[(4-Methylphenyl)sulfonyl]-3-(2-quinolinylmethoxy)benzeneethanamide or a pharmaceutically acceptable salt thereof.

5. N-[(4-Methylphenyl)sulfonyl]-3-(2-quinolinylmethoxy)benzamide or a pharmaceutically acceptable salt thereof.

6. A compound of Formula I as claimed in Claim 1 or Claim 2 which is in the form of a salt of an acid selected from hydrochloric, hydrobromic, sulfuric, nitric, phosphoric, methanesulphonic benzenesulphonic, acetic, citric, fumaric, maleic and succinic.

7. A compound of formula I or a pharmaceutically acceptable salt thereof as defined in any one of Claims 1, 2 or 6 for use as a pharmaceutical.

8. A pharmaceutical composition comprising a compound of formula I or a pharmaceutically acceptable salt thereof as claimed in any one of Claims 1, 2 or 6 and a pharmaceutically acceptable carrier.

9. A process for preparing a compound of formula I as defined in Claim 1 which comprises one of the following:

A) reacting a compound of formula

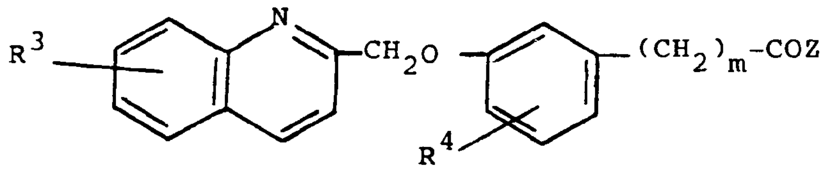

(II)

wherein m, $R^3$ and $R^4$ are as defined in Claim 1 and Z is OH or a reactive derivative thereof, with a sulphonamide of formula

$HNR^2SO_2R^1$ (III)

wherein $R^1$ and $R^2$ are as defined in Claim 1, to give a compound of formula I,

B) reacting a compound of formula (IV)

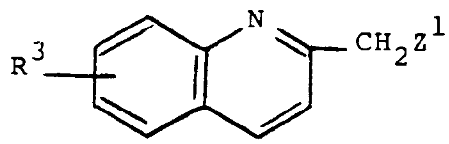

(IV)

wherein $R^3$ is as defined in Claim 1 and $Z^1$ represents halogen or an organic sulphonyloxy radical, with a compound of formula:

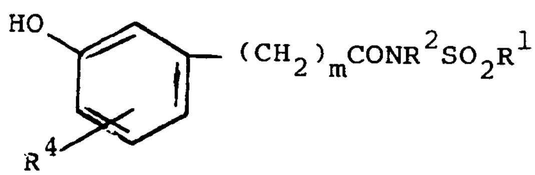

(Va)

wherein m, $R^1$, $R^2$ and $R^4$ are as defined in Claim 1 to give a compound of formula I,

C) converting a compound of formula I as defined in Claim 1 to an acid addition salt thereof by addition of an acid or basifying an acid addition salt to give a compound for formula I.

10. A compound of formula I or a pharmaceutically acceptable salt thereof as defined in any one of Claims 1 to 7 for use as an anti-inflammatory or anti-allergic agent.

## Patentansprüche

1. Verbindung der Formel

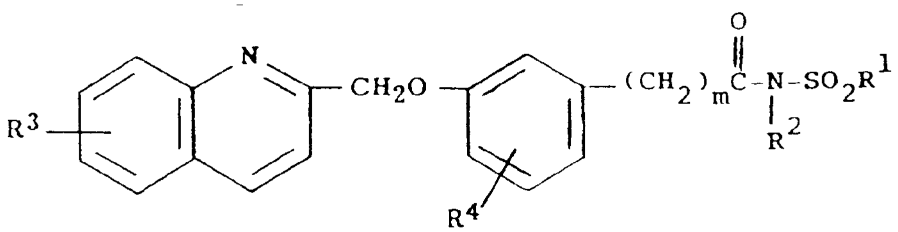

(I),

worin

$R^1$ Phenyl darstellt, das mit $R^5$ substituiert ist;

$R^2$ Wasserstoff oder $C_1$-$C_6$-Alkyl bedeutet;

$R^3$, $R^4$ und $R^5$ jeweils unabhängig Wasserstoff, $C_1$-$C_6$-Alkyl, Nitro, Trifluormethyl, Halogen, $C_1$-$C_6$-Alkoxy, ($C_1$-$C_6$-Alkoxy)carbonyl oder $C_2$-$C_7$-Alkanoyloxy darstellen; und

m 0 - 10 ist;

oder ein pharmazeutisch annehmbares Salz hievon.

2. Verbindung nach Anspruch 1, worin m 0, 1 oder 2 ist.

3. N-[(4-Methylphenyl)sulfonyl]-3-(2-chinolinylmethoxy)benzolpropanamid oder ein pharmazeutisch annehmbares Salz hievon.

4. N-[(4-Methylphenyl)sulfonyl]-3-(2-chinolinylmethoxy)benzolethanamid oder ein pharmazeutisch annehmbares Salz hievon.

5. N-[(4-Methylphenyl)sulfonyl]-3-(2-chinolinylmethoxy)benzamid oder ein pharmazeutisch annehmbares Salz hievon.

6. Verbindung der Formel (I) nach Anspruch 1 oder 2 in Form eines Salzes einer Säure, ausgewählt aus Chlorwasserstoff-, Bromwasserstoff-, Schwefel-, Salpeter-, Phosphor-, Methansulfon-, Benzolsulfon-, Essig-, Zitronen-, Fumar-, Malein- und Bernsteinsäure.

7. Verbindung der Formel (I) oder ein pharmazeutisch annehmbares Salz hievon, wie in einem der Ansprüche 1, 2 oder 6 definiert, zur Verwendung als Heilmittel.

8. Pharmazeutische Zusammensetzung umfassend eine Verbindung der Formel (I) oder ein pharmazeutisch annehmbares Salz hievon, wie in einem der Ansprüche 1, 2 oder 6 definiert, und einen pharmazeutisch annehmbaren Träger.

9. Verfahren zur Herstellung einer Verbindung der Formel (I) wie in Anspruch 1 definiert, umfassend eines des folgenden:

A) Umsetzen einer Verbindung der Formel

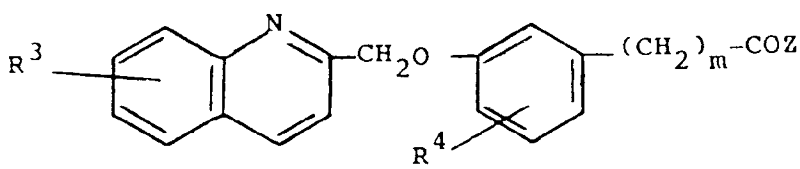

(II),

worin m, $R^3$ und $R^4$ wie in Anspruch 1 definiert sind, Z OH oder ein reaktives Derivat hievon darstellt, mit einem Sulfonamid der Formel

$$HNR^2SO_2R^1 \qquad (III),$$

worin $R^1$ und $R^2$ wie in Anspruch 1 definiert sind, wobei eine Verbindung der Formel (I) erhalten wird,

B) Umsetzen einer Verbindung der Formel (IV)

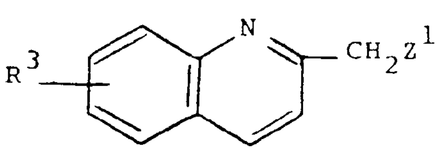

(IV),

worin $R^3$ wie in Anspruch 1 definiert ist und $Z^1$ Halogen oder einen organischen Sulfonyloxyrest darstellt, mit einer Verbindung der Formel

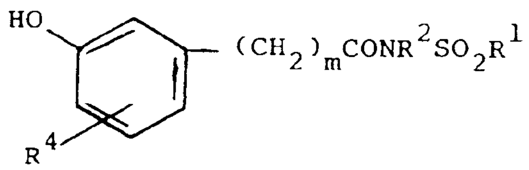

(Va),

worin m, $R^1$, $R^2$ und $R^4$ wie in Anspruch 1 definiert sind, wobei eine Verbindung der Formel (I) erhalten wird,

C) Überführen einer Verbindung der Formel (I) wie in Anspruch 1 definiert in ein Säureadditionssalz hievon durch Zugabe einer Säure oder basisch machen eines Säureadditionssalzes, wobei eine Verbindung der Formel (I) erhalten wird.

10. Verbindung der Formel (I) oder ein pharmazeutisch annehmbares Salz hievon wie in einem der Ansprüche 1 bis 7 definiert zur Verwendung als entzündungshemmendes oder anti-allergisches Mittel.

## Revendications

1. Composé de formule :

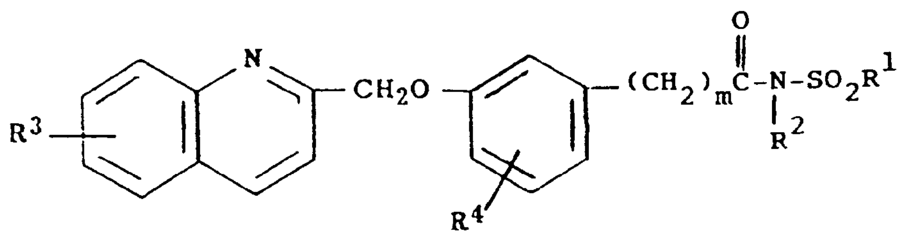

(I)

dans laquelle

$R^1$ est un groupe phényle substitué avec un radical $R^5$ ;

$R^2$ est l'hydrogène ou un groupe alkyle en $C_1$ à $C_6$ ;

$R^3$, $R^4$ et $R^5$ représentent chacun indépendamment l'hydrogène ou un radical alkyle en $C_1$ à $C_6$, nitro, trifluorométhyle, halogéno, alkoxy en $C_1$ à $C_6$, (alkoxy en $C_1$ à $C_6$)carbonyle ou alcanoyloxy en $C_2$ à $C_7$ ;

m a une valeur de 0 à 10 ;

ou un sel pharmaceutiquement acceptable de ce composé.

2. Composé suivant la revendication 1, dans lequel m a la valeur 0, 1 ou 2.

3. Le N-[(4-méthylphényl)sulfonyl]-3-(2-quinolinylméthoxy)benzènepropanamide ou un sel pharmaceutiquement acceptable de ce composé.

4. Le N-[(4-méthylphényl)sulfonyl]-3-(2-quinolinylméthoxy)benzène-éthanamide ou un sel pharmaceutiquement acceptable de ce composé.

5. Le N-[(4-méthylphényl)sulfonyl]-3-(2-quinolinylméthoxy)benzamide ou un sel pharmaceutiquement acceptable de ce composé.

6. Composé de formule I suivant la revendication 1 ou la revendication 2, qui est sous la forme d'un sel d'un acide choisi entre les acides chlorhydrique, bromhydrique, sulfurique, nitrique, phosphorique, méthanesulfonique, benzènesulfonique, acétique, citrique, fumarique, maléique et succinique.

7. Composé de formule I ou un sel pharmaceutiquement acceptable de ce composé tel que défini dans l'une quelconque des revendications 1, 2 ou 6, destiné à être utilisé comme composé pharmaceutique.

8. Composition pharmaceutique, comprenant un composé de formule I ou un sel pharmaceutiquement acceptable de ce composé suivant l'une quelconque des revendications 1, 2 ou 6 et un support acceptable du point pharmaceutique.

9. Procédé de préparation d'un composé de formule I suivant la revendication 1, qui comprend l'une des opérations suivantes :

A) réaction d'un composé de formule

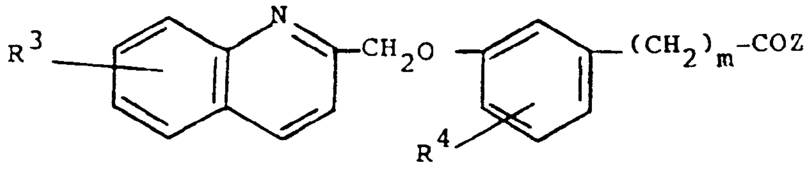

(II)

dans laquelle m, $R^3$ et $R^4$ sont tels que définis dans la revendication 1 et Z est un groupe OH ou un dérivé réactif de ce groupe, avec un sulfonamide de formule

$HNR^2SO_2R^1$ (III)

dans laquelle $R^1$ et $R^2$ sont tels que définis dans la revendication 1, pour obtenir un composé de formule I,

B) réaction d'un composé de formule (IV)

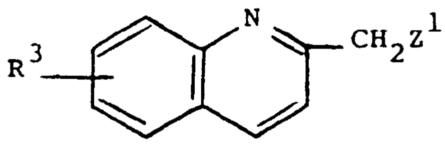

(IV)

dans laquelle $R^3$ est tel que défini dans la revendication 1 et $Z^1$ représente un halogène ou un radical sulfonyloxy organique, avec un composé de formule :

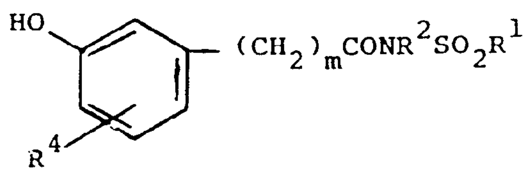

(Va)

dans laquelle m, $R^1$, $R^2$ et $R^4$ sont tels que définis dans la revendication 1, pour obtenir un composé de formule I,

C) transformation d'un composé de formule I tel que défini dans la revendication 1 en un sel d'addition d'acide de ce composé par addition d'un acide ou par alcalinisation d'un sel d'addition d'acide pour former un composé de formule I.

10. Composé de formule I ou sel pharmaceutiquement acceptable de ce composé tel que défini selon l'une quelconque des revendications 1 à 7, destiné à être utilisé comme agent anti-inflammatoire ou anti-allergique.